# EUROPEAN PATENT SPECIFICATION

(11) **EP 0 529 083 B1**
(45) Date of publication and mention of the grant of the patent: **18.12.1996**
(21) Application number: 92904407.1
(22) Date of filing: 10.02.1992
(51) Int. Cl.: C12P 17/02, C12N 5/04

(54) **PROCESS FOR PRODUCING TAXOL BY CELL CULTURE OF TAXUS SPECIES**
VERFAHREN ZUR HERSTELLUNG VON TAXOL MITTELS TAXUSSPEZIES-ZELLKULTUR
PROCEDE POUR PRODUIRE DU TAXOL PAR CULTURE CELLULAIRE DE L'ESPECE DE TAXUS

(30) Priority: 12.02.1991 JP 19010/91
(43) Date of publication of application: 03.03.1993
(73) Proprietor: Nippon Steel Corporation, Chiyoda-ku Tokyo 100 (JP)
(72) Inventor: SAITO, Koji, c/o Nippon Steel Corporation, Nakahara-ku, Kawasaki-shi, Kanagawa 211 (JP); OHASHI, Hiroaki, c/o Nippon Steel Corporation, Nakahara-ku, Kawasaki-shi Kanagawa 211 (JP); HIBI, Masaaki, c/o Nippon Steel Corporation, Nakahara-ku, Kawasaki-shi, Kanagawa 211 (JP); TAHARA, Makoto, c/o Nippon Steel Corporation, Nakahara-ku, Kawasaki-shi, Kanagawa 211 (JP)
(74) Representative: VOSSIUS & PARTNER
(86) International application number: JP9200132
(87) International publication number: WO9213961

(56) References cited:
- US-A- 5 019 504
- PROCEEDINGS OF THE 80th ANNUAL MEETING OF THE AMERICAN ASSOCIATION FOR CANCER RESEARCH, of 24-27 May 1989, San Francisco, CA (US), vol. 30, March 1989; A.A. CHRISTEN et al., p. 566, no. 2252/
- Plant Cell Report, Vol.12, p.479-482, (1993)
- J.Natl.Products, Vol.53, p.1609-1610, (1990)
- "In vitro culture of higher plants", p.71, Martinus Nijhoff Publishers (1987)
- "Plant cell culture, A practical approach", p.90, IRL-Press (1985)

## Description

The present invention relates to cell culture of plants belonging to the genus Taxus. More specifically, the present invention relates to a process for producing taxol by cell culture of plants belonging to the genus Taxus.

Taxol is an alkaloid of diterpene, which exhibits a carcinostatic activity and inhibits cytokinesis.

Taxol is a compound promising as an antitumor agent. In particular, for ovarian cancer, taxol has been subjected to the Phase III clinical trial sponsored by the National Cancer Institute (NCI) of National Institute of Health in the U.S.A. In the known process, taxol is produced by an extraction from the bark of the wild Pacific Yew tree, Taxus brevifolia NUTT. However, since the Pacific Yew plants grow very slowly, the regrowth of the bark after removal thereof is not expected, and further, the content of taxol therein, and the production efficiency thereof, are very low. For example, as much as 10 tons of the bark are required for producing as little as 1 kg of taxol. Accordingly, it is estimated that 2000 to 4000 Pacific Yew trees, which are 50 to 250 years old, must be cut down to yield 1 kg of taxol. Consequently, many companies and research institutes are attempting to develop alternative processes for producing taxol.

As a substitute for extracting the alkaloid from rare plant sources, there can be mentioned a process for extracting the desired alkaloid from cultured cells from the objective plant tissues grown in an artificial growth medium, but no example of a successful production and/or isolation of taxol from cultured cells of the plant belonging to the genus Taxus has been described in any literature. For example, M. A. Zenkteler et. al. (Acta. Soc. Bot. Pol., 39 (1): pp. 161-173, (1970)) disclose the formation of calli from female gametophytes of Taxus baccata LINN., but there is no description of whether or not the disclosed callus formation protocols can be applied to other species of the genus Taxus, and no description of whether or not the calli produce any alkaloid. Also there is no description teaching such matters. Nevertheless, research into the development of processes for producing taxol by cell culture has not been abandoned, since cell culture is still thought to be feasible as a process for producing same.

Abstract no. 2252 on p. 566 of vol. 30 of the Proceedings of the Eightieth Annual Meeting of the American Association of Cancer Research 24-27 May 1989 and US-A-5,019,504 both disclose a method for taxol production in which
tissue explants of Taxus brevifolia were placed on solid media;
the resulting cell lines were grown in suspension culture; and
taxol was obtained from the culture extract.

However, the described methods do not lead to an efficient production of taxol by the cultured cells.

Therefore, the object of the present invention is, to provide a process for efficiently producing taxol by cell culture of Taxus plant.

To attain the object described above, the present inventors have made intensive research into callus initiation from tissues of plants belonging to the genus Taxus and into proliferation of the callus cells. As a result, it has been found that cultured cells having a high taxol content can be efficiently obtained by culturing specific tissues originating from specific species belonging to the genus Taxus, whereby the present invention was achieved.

Namely, according to the present invention, there is provided a process for producing taxol by culturing cells originating from tissues of plants belonging to the genus Taxus, which comprises:
a) a step of preparing a living tissue;
b) a step of culturing the tissue obtained in step a) in a nutrient medium containing a gibberellin suitable for inducing a callus, to thereby induce a callus;
c) a step of culturing the callus cells obtained in step b) in a nutrient medium suitable for proliferating suspension culture cells; and
d) a step of recovering taxol from the cultured product obtained in step c).

Also provided are a process for inducing and proliterating the callus using some of the above-described steps, and the callus or suspension culture cells containing at least taxol as an alkaloid.

The phrase "plants belonging to the genus Taxus", as used in the present invention, means plants belonging to the genus Taxus of the family Taxaceae, and suitable for the object of the present invention (i.e. production of taxol). Typical examples thereof include, but are not restricted to, Pacific Yew (Taxus brevifolia NUTT.) and Japanese Yew (Taxus cuspidata SIE3. et ZUCC.).

According to the present invention, living tissues are prepared from specific tissues of these plants in a first stage. Consequently, as a piece of plant material put into culture, shoots, leaves, roots, flowers, fruits and seeds can be used as it is, or the plant materials may include those prepared in a form suitable for the culture, for example, in the case of the seeds, clean germinating tissues isolated from surface sterilized seeds. Of these leaves, shoots and female gametophytes, which are nutrient-storage tissues of a Gymnosperms seed, are preferable for the present invention. The endosperms or female gametophytes have been hitherto used for studies of tripoid or haploid plant development, and for studies of the bio-synthesis of seed storage nutrients. Surprisingly, the female gametophytes are found particularly suitable for initiating a callus and producing suspension culture cells containing considerable amounts of taxol. The above-described preparation is part of the concept of including treatments in which the plant materials for culture are isolated as they are and alive, and are surface sterilized. Typical examples of such preparations involve, but are not limited to, steps in which, first the materials collected from plants of the genus Taxus are sterilized with 70% ethanol and an aqueous solution of sodium hypochlorite, the raw materials are then aseptically divided into pieces, and thereafter, are transferred onto media solidified by agar or gellan gum suitable for inducing a callus as described hereinbelow.

The living tissues obtained in the above-described stage are then cultured on a nutrient media containing a gibberellin suitable for callus induction, and the calli thus derived are transferred to a nutrient medium suitable for the proliferation of callus cells. The primary components to be used in the nutrient medium include water; mineral nutrients, e.g. nitrogen (ammonium salts and nitrates), phosphorus, potassium, calcium, magnesium, sulfur, etc.; sugars, e.g., sucrose, glucose, fructose, maltose, etc.; organic substances such as vitamins and amino acids; naturally originating substances such as coconut milk; and optionally, gelling agents, e.g., agar, gellan gum, alginic acid, and agarose. The basic formulations of mineral nutrients known as basic media include Schenk & Hildebrandt medium (hereinafter referred to as "SH medium"), Murashige & Skoog medium (hereinafter referred to as "MS medium"), Gamborg's B5 medium, White's medium, Nitsch & Nitsch medium, Nagata & Takebe medium and woody plant medium. As regards media for proliferating callus cells a gibberellin (hereinafter referred to as "GA", such as GA1 and GA3) is added to these basic media and further optionally auxins such as 1-naphthaleneacetic acid (hereinafter referred to as "NAA"), indole-3-acetic acid (hereinafter referred to as "IAA"), indole-3-butyric acid (hereinafter referred to as "IBA"), and 2,4-dichlorophenoxyacetic acid (hereinafter referred to as "2,4-D") or cytokinins such as benzylaminopurine (hereinafter referred to as "BA"), zeatin, and 6-furfurylaminopurine (kinetin); are added as plant growth regulators. As the nutrient media suitable for inducing the callus according to the present invention, media in which a gibberellin and optionally auxins and/or cytokinins are added to the above-described basic media are preferable. Typically, a medium using the SH medium as the basic medium to which BA as the cytokinin and GA₃ as the gibberellin are added, is preferable. On the other hand, as the nutrient media suitable for proliferating suspension culture cells, the above-described basic media with cytokinins and auxins added thereto are used, with the media using the SH medium as the basic medium and having kinetin as the cytokinin and NAA or 2,4-D as the auxin added thereto being particularly preferable. The pH levels of such media are adjusted to 5 to 7, preferably 5.5 to 6.0, with an appropriate acid or alkali.

Each of the above-described cultures using these media can be carried out at a temperature in the range of 15-25°C with or without light-irradiation. In particular, the cultures for the proliferation of the callus cells are preferably carried out in a liquid medium, in which the medium containers are placed on a rotary shaker.

The isolation of taxol thus obtained from the cultured products can be carried out by following the method of extracting various alkaloids from cultured tissue followed by purification, which is known per se. The term "cultured products" used herein is intended to include calli, cultured cells, clumps of cultured cells, cultured tissue, cultured organs and a medium used for the culture. Although not restricted thereto, the taxol can be isolated from such cultivated products by separating calli or cultured cells from media, drying and pulverizing same, extracting taxol from the resulting powder with an appropriate organic solvent, optionally washing the organic phase with water, drying the organic solvent over anhydrous sodium sulfate, magnesium sulfate or calcium chloride, and then evaporating the solvent. If necessary, the taxol thus isolated can be purified by various chromatographic purifications or recrystallization, etc. Examples of an organic solvent which can be used in the extraction include chlorinated hydrocarbons such as methylene chloride and dichloroethane; and alcohols such as methyl, ethyl and isopropyl alcohol.

The substance isolated by the process described above has been confirmed to be taxol, by comparing the NMR, IR, MASS, and UV spectrums thereof with those of a standard sample of taxol received from the above-described NCI.

According to the present invention, the calli or cultured cells having a high taxol content can be effectively obtained by the cell culture of the plants belonging to the genus Taxus (for example, about 10 g of cells in dry matter was obtained from a liter of the suspension culture, and the cells thus obtained had a taxol content of 0.05%.) The taxol content of the dried callus was approximately 10 times greater than that of the dried bark of Pacific Yew trees.

The calli or suspension cells containing taxol may be used as an antitumor agent as are, by isolating and pulverizing same.

### EXAMPLES

The present invention will now be described in greater detail by referring to working examples 1, 3 and 4. Examples 2 and 5 are not within the scope of the claims.

### Example 1

A seed (5 g) of Taxus brevifolia NUTT from which aril had been removed was immersed in an aqueous solution of 70% W/W ethanol (50 mℓ) for 10 to 30 seconds, and then in an aqueous sodium hypochlorite solution having an effective chlorine concentration of 1% (50 mℓ) for 20 minutes, and there after, was rinsed three times in sterile water (100 mℓ) to eliminate surface contaminants. After the sterilization, the hard external seed coat and the thin internal seed coat were removed with tweezers, and resulting embryos and female gametophytes were then excised and used as an explant.

To prepare medium for culture of an explant obtained as described above, SH medium (supplemented with 0.25% by weight of gellan gum) containing 1, 5 or 10 mg/ℓ of BA as a growth regulator, and having a pH value adjusted to 5.8 with potassium hydroxide or hydrochloric acid, was sterilized by using an autoclave at temperature of 120°C for 15 minutes, and thereafter, GA₃ (separately sterilized by passing through a membrane filter) was added to concentrations of 0, 1, 10 or 100 mg/ℓ. The medium was dispensed into test tubes and solidified, in which the explants were transferred and cultured at a temperature of 20° or 25°C under a light (16 hours per day), to thus induce in vitro germination of an embryo or callus initiation. It was found that the callus was induced from female gametophytes after approximately 2 months of culture. The results of the callus induction are shown in Table 1.

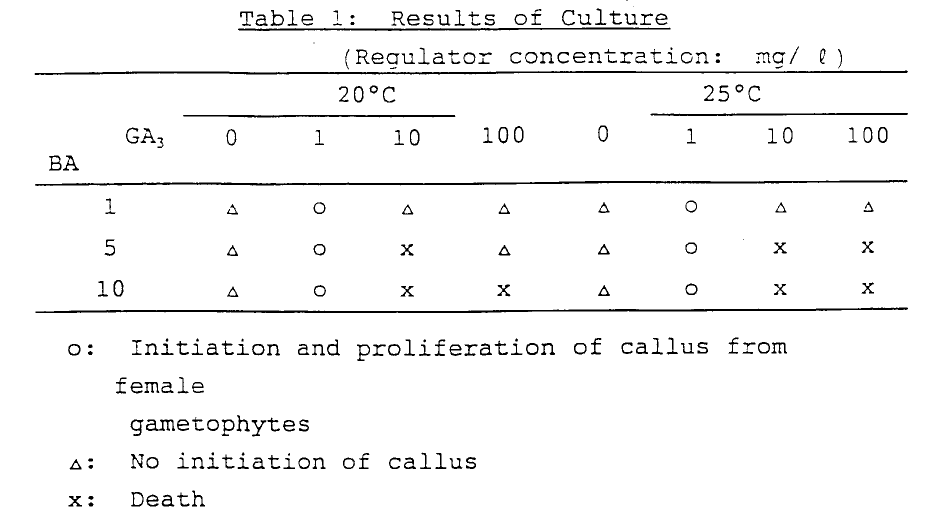

### Example 2

A leaf (5 g) and a shoot (5 g) of Taxus cuspidata SIEB. et ZUCC. were immersed in an aqueous solution of 70% W/W ethanol (50 mℓ) for 15 seconds, and then in an aqueous sodium hypochlorite solution having 1% of an effective chlorine concentration for 10 minutes, and there after, were rinsed three times with sterile water (100 mℓ) to eliminate surface contaminants. After the sterilization, the leaf and the shoot were cut to lengths of approximately 5 mm.

The explants thus prepared were transferred to MS medium (supplemented with 0.25% by weight of gellan gum) containing NAA and kinetin as plant growth regulators in the concentrations as shown in Tables 2 and 3, and having a pH value adjusted to 5.8 with potassium hydroxide or hydrochloric acid, and were then cultured at a temperature of 20° or 25°C in the dark or under a light (16 hours per day) to induce a callus. As shown in Table 4, the callus was induced at particular combinations of two plant growth regulator and sucrose concentrations.

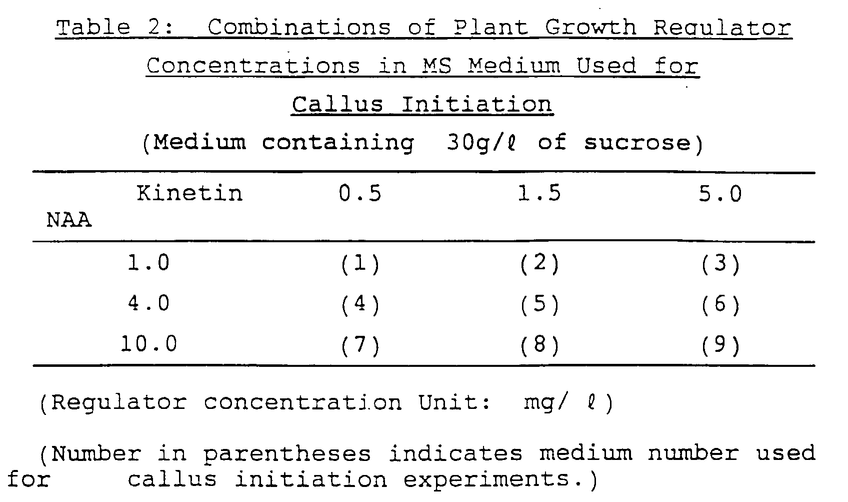

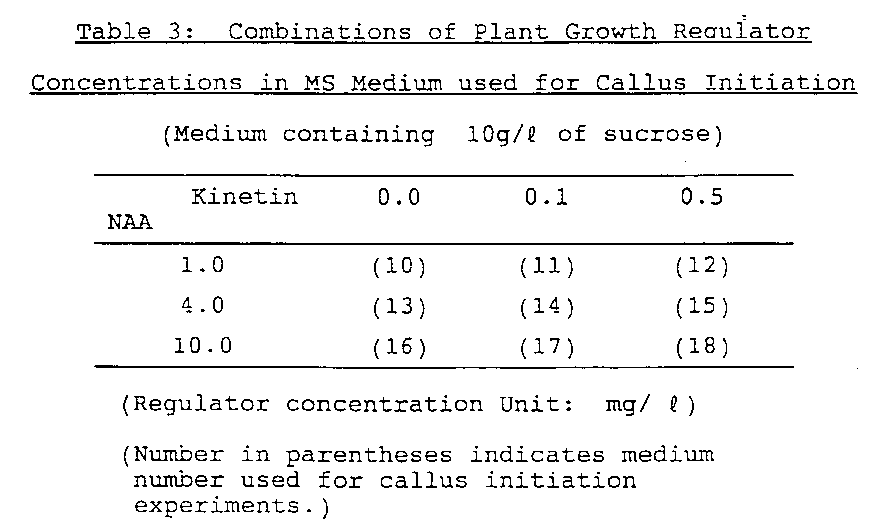

**Table 4:**

| Results of Callus Induction after 2 months of culture | | | | |
|---|---|---|---|---|
| Medium No. | Leaf | | Stem | |
| | Light | Dark | Light | Dark |
| (1) | x | x | o | △ |
| (2) | x | x | x | x |
| (3) | x | x | x | x |
| (4) | x | x | △ | x |
| (5) | x | x | o | x |
| (6) | x | x | △ | x |
| (7) | x | x | x | x |
| (8) | △ | x | x | x |
| (9) | x | x | x | x |
| (10) | x | x | △ | x |
| (11) | x | x | x | x |
| (12) | x | x | x | x |
| (13) | x | x | x | x |
| (14) | △ | x | o | x |
| (15) | x | x | o | x |
| (16) | x | x | x | x |
| (17) | △ | x | x | x |
| (18) | x | x | x | x |
| o: Initiation and proliferation of callus | | | | |
| △: No initiation of callus | | | | |
| x: Death | | | | |

### Example 3

The callus induced from female gametophytes in Example 1 was transferred to SH medium (liquid medium) containing 5 mg/ℓ of NAA and 0, 0.1 or 5 mg/ℓ of kinetin as plant growth regulators and having a pH value adjusted to 5.8. Erlenmeyer 100 mℓ flasks each containing 40 mℓ of the medium were used for the culture. The flasks were placed on a rotary shaker and shaken at a rate of 100 rpm, and at a temperature of 20°C, in the dark.

After 4 weeks of culture, it was observed that, in the medium containing 5 mg/ℓ of NAA alone as a growth regulator, the cell mass increased by about 5 times.

Cell growth in the medium containing 5 mg/ℓ of NAA was examined at a temperature of 20° or 25°C in the dark or under a light (16 hours per day). Although cell mass increased under all test conditions, the largest growth rate was observed at a temperature of 20°C in the dark.

When 2,4-D was used as the auxin in place of NAA, similar results were obtained.

### Example 4

The cells grown in Example 3 were collected and air-dried. The extraction and purification of taxol from the cells were carried out with reference to a process for extraction of taxol from the bark of a Taxus plant (M. Keith, J. Natural Products, Vol. 53 (5), pp. 1249-1255, 1990). The air-dried cells were pulverized, and extracted with methylene chloride-methanol (1 : 1). The extraction solution was separated from the cell debris and evaporated, and the residue was then suspended in methylene chloride. This organic suspension was washed with water, and the solvent was then evaporated. The resulting residue was resuspended in methanol, and taxol was isolated and purified from the methanol suspension by High Performance Liquid Chromalography (HPLC) equipped with a reverse phase column (M. Keith, J. Liquid Chromatography, Vol. 12, pp. 2117-2132, 1989).

The identification of taxol from the cell culture was carried out by comparing a retention time of peaks obtained for the above-mentioned methanol suspension by HPLC with that of a standard sample of taxol from NCI, and as a result, a peak with an identical retention time with the standard sample was observed. The chemical structure of the substance purified from the methanol suspension by HPLC was determined by a mass spectrum analysis and NMR, and it was confirmed that the substance was taxol. The taxol content was 0.05% of dry matter of suspension cells. This content was larger than that of dried bark by approximately 10 times. (M. Keith, J. Natural Products, Vol. 53 (5), pp. 1249-1255, 1990).

### Example 5

The callus induced from the shoot in Example 2 was transferred to SH medium (liquid medium) containing 5 mg/ℓ of NAA and 0, 0.1, or 5 mg/ℓ of kinetin as plant growth regulators and having a pH value adjusted to 5.8. Erlenmeyer 100 mℓ flasks each containing 40 mℓ of the medium were used for the culture. The flasks were placed on a rotary shaker and shaken at a rate of 100 rpm, and at a temperature of 20°C, in the dark.

After 3 weeks of culture, the callus cells were proliferated only in the medium containing 5 mg/ℓ of NAA alone, at a temperature of 20°C in the dark.

### INDUSTRIAL APPLICABILITY

According to the present invention, callus cells of the Taxus plants containing at least taxol as alkaloids can be effectively induced and proliferated with artificial growth media. Furthermore, taxol can be isolated from these cultured cells. Accordingly, the present invention is available for use in the field of manufacturing medicinal preparations possessing a carcinostatic activity.

## Claims

1. A process for producing taxol by culturing cells originating from tissues of plants belonging to the genus Taxus, which comprises:
a) a step of preparing a living tissue;
b) a step of culturing the tissue obtained in stage a) in a nutrient medium containing gibberellin and suitable for inducing a callus, to thereby induce a callus;
c) a step of culturing the callus cells obtained in step b) in a nutrient medium suitable for proliferating suspension culture cells ; and
d) a step of recovering taxol from the cultured products obtained in step c).

2. The process according to claim 1, wherein the plant species belonging to genus Taxus is Taxus brevifolia NUTT.

3. The process according to claim 1, wherein the plant belonging to genus Taxus is Taxus cuspidata SIEB. et ZUCC.

4. The process according to any one of claims 1 to 3, wherein the tissue is selected from the group consisting of leaves, shoots and female gametophytes.

5. The process according to any one of claims 1 to 4, wherein the cultivated product is suspension culture cells.

6. The process according to any one of claims 1 to 5, wherein the nutrient medium suitable for inducing the callus is SH medium to which benzyladenine and gibberellin have been added as plant growth regulators.

7. The process according to any one of claims 1 to 6, wherein the nutrient medium suitable for proliferating the suspension culture cells is SH medium to which the combination of kinetin with naphthaleneacetic acid or of kinetin with 2,4-dichlorophenoxyacetic acid has been added as plant growth regulators.

8. A process for inducing a callus and proliferating suspension culture cells comprising steps of a) to c) of claim 1, a female gametophyte of Taxus brevifolia NUTT. or Taxus cuspidata SIEB. et ZUCC. being used as the living tissue.

9. A callus or suspension culture cells obtained by the process of claim 8, which contains at least taxol as alkaloids.

## Patentansprüche

1. Verfahren zur Herstellung von Taxol durch Züchtung von Zellen, die aus Gewebe von Pflanzen stammen, die zur Gattung Taxus gehören, umfassend die Schritte:
a) Herstellung eines lebenden Gewebes;
b) Züchtung des Gewebes aus a) in einem Nährmedium, das Gibberellin enthält und für die Induktion von Callus geeignet ist, um Callus zu induzieren;
c) Züchtung der Calluszellen von b) in einem Nährmedium, das für die Vermehrung einer Suspensionszellkultur geeignet ist; und
d) Gewinnung von Taxol aus den gezüchteten Produkten von c).

2. Verfahren nach Anspruch 1, wobei die Pflanzenart, die zur Gattung Taxus gehört, Taxus brevifolia NUTT. ist.

3. Verfahren nach Anspruch 1, wobei die Pflanze, die zur Gattung Taxus gehört, Taxus cuspidata SIEB, et ZUCC. ist.

4. Verfahren nach einem der Ansprüche 1 bis 3, wobei das Gewebe ausgewählt ist aus Blättern, Sprossen und weiblichen Gametophyten.

5. Verfahren nach einem der Ansprüche 1 bis 4, wobei das gezüchtete Produkt eine Suspensionszellkultur ist.

6. Verfahren nach einem der Ansprüche 1 bis 5, wobei das Nährmedium, das für die Callusinduktion geeignet ist, SH-Medium ist, zu welchem Benzyladenin und Gibberellin als Pflanzen-Wachstumsregulatoren zugegeben wurden.

7. Verfahren nach einem der Ansprüche 1 bis 6, wobei das Nährmedium, das für die Vermehrung der Suspensionszellkultur geeignet ist, SH-Medium ist, zu welchem eine Kombination von Kinetin mit Naphthalinessigsäure oder Kinetin mit 2,4-Dichlorphenoxyessigsäure als Pflanzen-Wachstumsregulatoren zugegeben wurde.

8. Verfahren zur Induktion von Callus und zur Vermehrung einer Suspensionszellkultur, umfassend die Schritte a) bis c) von Anspruch 1, eine weibliche Gametophyte von Taxus brevifolia NUTT, oder Taxus cuspidata SIEB. et ZUCC., die als das lebende Gewebe verwendet werden.

9. Callus oder Suspensionszellkultur, erhalten durch das Verfahren nach Anspruch 8, welche zumindest Taxol als Alkaloid enthalten.

## Revendications

1. Procédé pour produire du taxol par culture de cellules provenant de tissus de végétaux appartenant au genre Taxus, qui comprend :
a) une étape de préparation d'un tissu vivant ;
b) une étape de culture du tissu obtenu dans l'étape a) dans un milieu nutritif contenant une gibbérelline et convenant pour induire un cal, pour induire ainsi un cal ;
c) une étape de culture des cellules de cal obtenues dans l'étape b) dans un milieu nutritif convenant pour faire proliférer des cellules en culture en suspension ; et
d) une étape de récupération du taxol à partir des produits cultivés obtenus dans l'étape c).

2. Procédé selon la revendication 1, dans lequel l'espèce végétale appartenant au genre Taxus est Taxus brevifolia NUTT.

3. Procédé selon la revendication 1, dans lequel le végétal appartenant au genre Taxus est Taxus cuspidata SIEB et ZUCC.

4. Procédé selon l'une quelconque des revendications 1 à 3, dans lequel le tissu est choisi dans le groupe comprenant les feuilles, les pousses et les gamétophytes femelles.

5. Procédé selon l'une quelconque des revendications 1 à 4, dans lequel le produit cultivé est constitué par des cellules en culture en suspension.

6. Procédé selon l'une quelconque des revendications 1 à 5, dans lequel le milieu nutritif convenant pour induire le cal est du milieu SH auquel de la benzyladénine et une gibbérelline ont été ajoutées comme régulateurs de croissance des végétaux.

7. Procédé selon l'une quelconque des revendications 1 à 6, dans lequel le milieu nutritif convenant pour faire proliférer les cellules en culture en suspension est du milieu SH auquel la combinaison de kinétine et d'acide naphtalène-acétique ou de kinétine et d'acide 2,4-dichlorophénoxyacétique a été ajoutée comme régulateur de croissance des végétaux.

8. Procédé pour induire un cal et faire proliférer des cellules en culture en suspension comprenant les étapes a) à c) de la revendication 1, un gamétophyte femelle de Taxus brevifolia NUTT. ou de Taxus cuspidata SIEB. et ZUCC. étant utilisé comme tissu vivant.

9. Cal ou cellules en culture en suspension obtenu(es) par le procédé selon la revendication 8, contenant au moins du taxol comme alcaloïde.
